# EUROPÄISCHE PATENTANMELDUNG

(11) **EP 0 592 785 A2**
(43) Veröffentlichungstag der Anmeldung: **20.04.1994**
(21) Anmeldenummer: 93112782.3
(22) Anmeldetag: 10.08.1993
(51) Int. Cl.: C12N 1/18, A23L 1/01, A23L 1/22

(54) **Hefen mit hohem natürlichem Glutaminsäure-Gehalt**

(30) Priorität: 10.10.1992 DE 4234240
(71) Anmelder: HÜLS AKTIENGESELLSCHAFT, D-45764 Marl (DE)
(72) Erfinder: Hill, Frank, Dr., D-40822 Mettmann-Oberschwarzbach (DE)

(57) **Zusammenfassung**

Gegenstand der Erfindung sind Hefen der Gattung Saccharomyces cerevisiae, die einen intrazellulären Gehalt an freier Glutaminsäure von 4 bis 15 %, bezogen auf die Trockensubstanz, aufweisen. Diese glutaminsäure-reichen Hefen kann man durch Mutation und Selektion in Gegenwart von Antagonisten der Glutaminsäure erhalten.

## Beschreibung

Die Erfindung betrifft Hefen der Gattung Saccharomyces cerevisiae mit einem hohen Gehalt an freier Glutaminsäure.

Mono-natrium-glutaminat, im folgenden als Natriumglutamat bezeichnet, ist ein natürlicher Geschmacksverstärker, der in vielen Lebensmitteln vorkommt. Die Bedeutung dieses Würzstoffes für einen vollen und runden Geschmack ist so groß, daß einer Reihe industriell hergestellter Lebensmittel chemisch reines Natriumglutamat zugesetzt wird. Dies ist bei einigen Produkten zulässig. Der Zusatz muß aber bei Produkten, die für den Endverbrauch bestimmt sind, deklariert werden.

Ein Einsatz von Bäckerhefe mit einem hohen natürlichen Glutaminsäure-Gehalt braucht dagegen nicht deklariert zu werden. Doch der Gehalt an freier Glutaminsäure in der Biomasse von Hefen liegt üblicherweise bei nur ca. 2 % der Trockensubstanz. Das geht beispielsweise aus Arch. Microbiol. (1981) 129, 368 - 70, hervor. Durch enzymatische oder chemische Hydrolyse der zelleigenen Peptide und Proteine kann der Gehalt bis auf etwa 4 % der wasserlöslichen Trockensubstanz gesteigert werden. Die Verdoppelung des Gehaltes ergibt sich dadurch, daß bei diesen Hydrolyseverfahren etwa 50 % der eingesetzten Hefebiomasse solubilisiert werden.

Die Glutaminsäure-Mengen bekannter Hefen sind für viele Einsatzzwecke in Lebensmitteln zu gering.

Aufgabe der vorliegenden Erfindung ist es daher, Hefen mit einem hohen natürlichen Glutaminsäure-Gehalt bereitzustellen.

Die Aufgabe wird erfindungsgemäß durch Hefen der Gattung Saccharomyces cerevisiae gelöst, die einen intrazellulären Gehalt an freier Glutaminsäure von 4 bis 15 %, bezogen auf die Trockensubstanz, aufweisen.

Der Glutaminsäure-Gehalt liegt vorzugsweise bei 9 bis 15 % der Trockensubstanz. Dabei wird unter Trockensubstanz die trockene Hefebiomasse verstanden.

Bei Hefen der Gattung Saccharomyces cerevisiae handelt es sich vorzugsweise um Hefestämme, die gegen Antagonisten der Glutaminsäure resistent sind. Das betrifft sowohl synthetische als auch natürliche Antagonisten. Beispiele dafür sind vor allem 3-Chloralanin, Diazo-oxo-norleucin, Azaserin und Phosphinotricin. Geeignet ist unter anderem aber auch γ-Glutamylhydrazid.

Der Hefestamm Saccharomyces cerevisiae DSM 7244 wird ganz besonders bevorzugt.

Glutaminsäure-akkumulierende Stämme werden sowohl unter haploiden Hefen als auch bei diploiden Hefen mit Resistenz gegen Antagonisten der Glutaminsäure gefunden. Diploide Hefen werden typischerweise in praktisch genutzen Verfahren der Lebensmitteltechnik eingesetzt. Die erfindungsgemäßen Hefen erhält man beispielsweise nach einem Verfahren, das aus den folgenden drei Schritten besteht:
a) Herstellung der Resistenzmutanten gegen einen Antagonisten der Glutaminsäure
b) Auswahl der glutaminsäure-akkumulierenden Stämme
c) Anzucht der ausgewählten Stämme unter gegebenenfalls modifizierten Bedingungen

Auf diese Art lassen sich glutaminsäure-reiche Spezialhefen mit begrenztem Aufwand herstellen. Der lösliche "Pool" der Glutaminsäure ist bei diesen resistenten Zellen gegenüber den nicht-resistenten Zellen des Wildtyps mindestens verdoppelt und kann durch veränderte Anzuchtbedingungen weiter gesteigert werden. Bei einem besonders vorteilhaften Anzucht-Verfahren wird ein Temperatur-Shift von +3 bis +6 °C sowie ein pH-Shift von 1 bis 4 pH-Einheiten eingestellt.

Die Hefen weise beispielsweise einen Gehalt von etwa 10 % freier Glutaminsäure in der Trockensubstanz auf. In Hefeextrakten, die aus diesen Hefebiomassen durch enzymatische Hydrolyse oder durch Lösemittel und/oder temperatur-induzierte Autolyse gewonnen werden, werden dann etwa 20 % freie Glutaminsäure, bezogen auf die wasserlösliche Trockensubstanz, erhalten. Dabei ist die wasserlösliche Trockensubstanz die trockene, wasserlösliche Hefebiomasse.

Mit den erfindungsgemäßen Hefen mit dem hohen intrazellulären Gehalt an freier Glutaminsäure können Extrakte sowie feste und flüssige Lebensmittel hergestellt werden. Durch die Hefen kann die Anreicherung von Lebensmitteln mit Natriumglutamat auf natürliche Art und Weise, ohne den Zusatz von chemisch reinem Natriumglutamat, erfolgen.

Derartige Lebensmittel sind beispielsweise Brot und andere Backwaren, alkoholische Getränke, Cracker, Brotaufstriche sowie verschiedene diätische Lebensmittel. Andere Lebensmittel, die mit einem Zusatz von Extrakten der glutaminsäure-reichen Hefen ("Hefextrakt") hergestellt werden, sind Suppen und Soßen, Konserven sowie Fleisch- und Wurstwaren.

Bei der Herstellung der glutaminsäure-reichen Hefen werden Hefen der Gattung Saccharomyces cerevisiae zuerst mutiert, wobei die Mutation beispielsweise durch UV-Licht erfolgen kann. Anschließend werden die überlebenden Zellen auf einem Nähragar unter Zusatz des Antagonisten der Glutaminsäure angezogen, worauf der Gehalt an Glutaminsäure chromatographisch bestimmt wird.

Die folgenden Beispiele sollen die Erfindung erläutern.

### Beispiel 1

Der Backhefestamm Saccharomyces cerevisiae A₂W₅ (Ohly GmbH, D-4370 Marl) wird mit ultraviolettem Licht behandelt. 10⁵ bis 10⁶ der überlebenden Zellen werden auf einen Minimalnähragar (Yeast nitrogen base w/o amino acids, Fa. Difco, Detroit, Michigan, USA) mit Zusätzen von 1 % Glycerin, 2 % Agar und 500 µg/ml 3-Chloralanin plattiert und bei 32 °C inkubiert. Nach 8 Tagen werden die gewachsenen Resistenzkolonien abgeimpft und durch Ausstrich gereinigt. Zur Ermittlung der Stämme mit einem hohen intrazellulären Gehalt an freier Glutaminsäure werden die Stämme in einer Nährlösung aus 3 % Melasse und 0,5 % Diammoniumhydrogen-phosphat bei pH 6 und 35 °C vermehrt. Die gewachsenen Zellen werden gewaschen und in destilliertem Wasser suspendiert. Die freien intrazellulären Aminosäuren werden aus den Zellen durch Erhitzen für 5 min auf 100 °C extrahiert. Nach dem Zentrifugieren wird der Gehalt an Aminosäuren in dem klaren Extrakt nach Umsetzung mit o-Phthaldialdehyd in einem Hochdruck-Flüssigkeit-Chromatographen bestimmt. Die chloralanin-resistente Mutante Saccharomyces cerevisiae DSM 7244 hat einen Gehalt von 41 mg/g Glutaminsäure in der Trockensubstanz.

Zum Vergleich weist der Wildtyp unter diesen Bedingungen einen Gehalt an extrahierbarer Glutaminsäure von nur 19 mg/g Trockensubstanz auf.

### Beispiel 2

Die ausgewählte Mutante Saccharomyces cerevisiae DSM 7244 wird im 10-1-Fermenter unter simulierten technischen Anzuchtbedingungen vermehrt. Dazu werden in 4 l Trinkwasser 1,5 g MgSO₄·7H₂O, 20 g KH₂PO₄, 40 g (NH₄)₂SO₄, 0,1 g ZnSO₄·7H₂O, 6 ml Entschäumer (Polypropylenglykol) und 2 mg D-Biotin gelöst. Der Ansatz wird mit 500 ml einer gewaschenen Zellsuspension des Stammes Saccharomyces cerevisiae DSM 7244 (mit einem Trockensubstanzgehalt von 60 g) beimpft. Es wird mit 600 UpM gerührt, mit 6 l/min Luft belüftet und die Temperatur bei 32 °C gehalten. Der pH-Wert wird auf 5,5 eingestellt. Diesem Ansatz werden in 14 h kontinuierlich 2 l Rübenmelasselösung mit 1 kg Melasse sowie 650 ml wäßrige Ammoniaklösung mit 90 ml 25%igem NH₃ zugesetzt. Die Geschwindigkeit der Zugabe ist so bemessen, daß in der Kultur kein Ethanol gebildet wird. Nach 14 h wird der pH-Wert durch Zugabe von Na₂CO₃ auf 7,0 eingestellt, die Temperatur auf 37 °C angehoben und 200 ml Glucoselösung mit 70 g Glucose innerhalb von 3 h kontinuierlich zugesetzt. Dann wird die Fermentation beendet, die Hefezellen werden zentrifugiert und zweimal mit Trinkwasser gewaschen.

Ausbeute: 350 g Trockensubstanz mit einem Gehalt von 10,0 % an freier Glutaminsäure.

### Beispiel 3

Mit der glutaminsäure-reichen Hefe aus Beispiel 2 werden Brötchen hergestellt. Die Teigeinwaage ist 141 g je Brötchen (86 g Weizenmehl, 1,2 g Kochsalz, 50 ml Wasser, 4 g feuchte Hefe mit 29 % Trockensubstanz). Nach einer Gärzeit von 30 min werden die Brötchen bei 220 °C gebacken. Nach dem Abkühlen werden die Backwaren verkostet. Brötchen, die mit der glutaminsäure-reichen Hefe bereitet wurden, schmeckten deutlich würziger als entsprechende Brötchen, bei denen statt der glutaminsäure-reichen Hefe eine handelsübliche Bäckerhefe verwendet wurde.

### Beipsiel 4

Mit der glutaminsäure-reichen Hefe aus Beispiel 2 wird ein Hefeextrakt bereitet. Dazu wird 1 l Hefesuspension mit 140 g Trockensubstanz mit 20 ml Ethylacetat versetzt und 21 h bei 55 °C geschüttelt. Dann wird abzentrifugiert und das Sediment einmal mit Wasser gewaschen. Die klaren Überstände werden vereinigt und gefriergetrocknet. Es werden 56 g Hefeextrakt erhalten. Der Gehalt an freier Glutaminsäure beträgt 19 % in der wasserlöslichen Trockensubstanz.

### Beispiel 5

Eine flüssige Speisewürze wird mit der glutaminsäure-reichen Hefe aus Beispiel 2 bereitet. Dazu wird in Wasser suspendierte Hefe (1 l mit 140 g Trockensubstanz) bei 37 °C gerührt. Der pH-Wert wird mit NaOH auf 13 eingestellt. Nach 2 h wird der pH-Wert mit HCl auf 7 eingestellt und ein flüssiges Proteasepräparat (COROLASE LS, Fa. Röhm, D-6100 Darmstadt) zugesetzt. Es wird für 4 h auf 55 °C erwärmt und gerührt. Dann wird ein flüssiges Phosphodiesterase-Präparat zugesetzt und weitere 12 h bei 55 °C gerührt. Danach wird abzentrifugiert und der klare Überstand durch Einengen am Rotationsverdampfer auf 15 % gelöste Trockensubstanz gebracht. Dann werden 15 % NaCl zugesetzt, der pH-Wert mit HCl auf 4,8 eingestellt und blank filtriert.

Die hellbraune Lösung hat einen neutralen Geruch und einen exzellenten, würzigen Geschmack.

## Patentansprüche

1. Hefen der Gattung Saccharomyces cerevisiae mit einem intrazellulären Gehalt an freier Glutaminsäure von 4 bis 15 %,
bezogen auf die Trockensubstanz.

2. Hefen nach Anspruch 1,
dadurch gekennzeichnet,
daß sie gegen Antagonisten der Glutaminsäure resistent sind.

3. Hefen nach Anspruch 2,
dadurch gekennzeichnet,
daß sie gegen 3-Chloralanin, Diazo-oxo-norleucin, Azaserin oder Phosphinotricin resistent sind.

4. Hefe nach Anspruch 3,
dadurch gekennzeichnet,
daß sie aus Saccharomyces cerevisiae DSM 7244 besteht.

5. Verfahren zur Herstellung von Hefen nach Anspruch 1,
dadurch gekennzeichnet,
daß man Hefen der Gattung Saccharomyces cerevisiae mutiert und dann mit Antagonisten der Glutaminsäure behandelt.

6. Verwendung von Hefen nach Anspruch 1 zur Herstellung von Extrakten sowie von festen und flüssigen Lebensmitteln.

7. Verwendung nach Anspruch 6 zur Herstellung von Backwaren und Speisewürzen.
